# EUROPEAN PATENT SPECIFICATION

(11) **EP 1 871 342 B1**
(45) Date of publication and mention of the grant of the patent: **13.12.2017**
(21) Application number: 06728418.2
(22) Date of filing: 21.03.2006
(51) Int. Cl.: A61K 9/127, A61K 31/05

(54) **A NOVEL INTER AND INTRA MULTILAMELLAR VESICULAR COMPOSITION.**
NEUE INTER- UND INTRAMULTILAMELLARE VESIKULÄRE ZUSAMMENSETZUNG
NOUVELLE COMPOSITION VESICULAIRE INTER ET INTRA MULTILAMELLAIRE

(30) Priority: 21.03.2005 IN DE06102005
(43) Date of publication of application: 02.01.2008
(73) Proprietor: Lifecare Innovations Pvt. Ltd., Haryana 122002 (IN)
(72) Inventor: KATARE, Om Prakash, Panjab University Campus, Sector 14,Chandigarh 160014 (IN); AGARWAL, Ravindra, Panjab University Campus, Sector 14,Chandigarh 160014 (IN); KUMAR, Bhushan, Panjab University Campus, Sector 14,Chandigarh 160014 (IN)
(74) Representative: Studio Torta S.p.A.
(86) International application number: PCT/IN2006/000113
(87) International publication number: WO 2006/100692

(56) References cited:
- EP-A1- 1 364 642
- WO-A1-94/07466
- US-A- 4 367 224
- US-A- 5 476 664
- US-A1- 2002 102 298
- US-A1- 2003 219 465
- R AGARWAL ET AL: "Preparation and in vitro evaluation of liposomal/niosomal delivery systems for antipsoriatic drug dithranol", INTERNATIONAL JOURNAL OF PHARMACEUTICS, vol. 228, no. 1-2, 1 October 2001 (2001-10-01), pages 43-52, XP55036053, ISSN: 0378-5173, DOI: 10.1016/S0378-5173(01)00810-9
- GEHRING W ET AL: "ENHANCEMENT OF THE PENETRATION OF DITHRANOL AND INCREASE OF EFFECT OF DITHRANOL ON THE SKIN BY LIPOSOMES//FOERDERUNG DER PENETRATION VON DITHRANOL UND POTENZIERUNG DES DITHRANOL-EFFEKTES DURCH LIPOSOMEN", ARZNEIMITTEL FORSCHUNG. DRUG RESEARCH, ECV EDITIO CANTOR VERLAG, AULENDORF, DE, vol. 42, no. 7, 1 January 1992 (1992-01-01), pages 983-985, XP008020889, ISSN: 0004-4172

## Description

### Field of Invention:

This invention relates to the field of drug delivery wherein the drug molecules are accommodated at various levels within the system (encapsulated or non-encapsulated) so that their transport to the target site is effected in a most desirable manner. The delivery system is structured by selecting the appropriate components of varied physico-chemical nature and designing them in an architectural pattern which suits for the purpose. This invention is for dermal problems like psoriasis where the delivery of the drug dithranol is desired at the epidermal germinal layer by way of generating microstructures in-situ with the help of bio-friendly and most suitable components.

### Background of invention:

Dithranol is the drug of choice for the topical treatment of psoriasis and many other skin afflictions. The drug is preferred over other therapeutic options in psoriasis like corticosteroids because of prolonged remission obtained with dithranol (relapse rate is very low) whilst the corticosteroids are associated with many serious side effects at local and systemic level i.e. rapid recurrences, skin atrophy and generalized immuno-suppression. It is the drug of choice in stable plaque psoriasis, which is the most prevalent form of psoriasis. It is also used in many other skin afflictions such as chronic eczemas, dermatophytoses, alopecia areata, and seborrheic dermatitis etc. But at the same time, the dithranol molecule is not patient friendly because of high irritation, staining of skin and clothes and perilesional inflammation. These drawbacks are associated with the inherent chemical and physicochemical characteristics of the drug i.e. chemical reactivity leading to degradation (i.e. auto-oxidation chain reaction resulting in formation of free radicals and oxidized products), imbalanced solubility (i.e. insoluble in water and highly soluble in organic solvent) and improper partitioning and permeability. And, so far made efforts to tackle these problems by way of chemical modification of the molecule or vehicular modification by using the conventional formulation approach (use of additives and oily base or creams) have met with a little success.

Dithranol has a long historical record of being the oldest and most efficacious topical drugs in the treatment of psoriasis (Squire, 1877, Shroot ct al., 1981). It traces its origin in the yellowish powder extract derived from a legume tree, *Andira araroba* that was used by aborigines of Brazil for the treatment of mycoses of the skin. The active principle was found to be chrysarobin. It was exported from Brazil to Goa in India, where it got the name Goa powder, which was found to have a good therapeutic effect in psoriasis (reported by Squire in 1877). During World War I, the trade with Brazil was disrupted and supply of chrysarobin was scarce. This scarcity eventually led to the development of synthetic version of the drug (synthesized from purgative compound Isitizin, 1,8-dihydroxy anthraquinone). The chemical factory Bayer developed a synthetic substitute of chrysarobin, 1,8-dihydroxy-9-anthrone, which was named Cignolin. Cignolin was later called Anthralin in United States and dithranol in Europe. In 1916, Eugen Galewsky introduced dithranol as a drug for the treatment of psoriasis.

### Dithranol profile

| | | |
|---|---|---|
| Chemical name | : | 1,8-dihydroxyanthrone |
| Empirical formula | : | C₁₄H₁₀O₃ |
| Structural formula | : | |
| | | 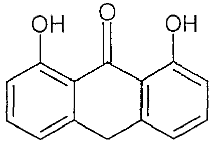 |
| CAS registry number | : | 1143-38-0 |
| Molecular weight | : | 226.23 |
| Description | : | yellow to orange yellow or yellowish brown, odorless or almost odorless crystalline powder |
| Melting point | : | 178-181°C |
| Solubility | : | Practically insoluble in water, slightly soluble in alcohol, ether, glacial acetic acid, sparingly soluble in acetone, soluble in methylene chloride, chloroform, dissolves in dilute solution of alkali hydroxides. |

### Pharmacokinetics and pitarmacodynamics (Muller 1996, Reichert et al., 1985, Shroot et al., 1992):

Topically, dithranol penetrates into the skin with an impaired stratum corneum barrier in 30 minutes and the highest concentration of unchanged dithranol is found in the horny layer. But in certain forms of psoriasis dithranol finds difficulty to penetrate and so salicylic acid is incorporated as a keratolytic agent (for improved penetration). Dithranol is known to exert its antipsoriatic activity by virtue of more than one modes of action. It acts at various cellular, intracellular and intercellular levels, which includes nucleic acids, various biochemical pathways, and immune-mediated reactions etc. to bring about therapeutic efficacy. It inhibits keratinocyte proliferation and results in normalization of keratin expression. Epidermal cell turnover rate is decreased by dithranol resulting in alleviation of the symptoms of the disease.

It also has a potent effect on cellular respiration. Target organelle for dithranol is mitochondria where it accumulates and inhibits cellular respiration by its interaction with the electron transport chain, resulting finally in a reduction of ATP synthesis. The lowering of cellular energy supply results in inhibition of energy dependent process such as DNA replication which slows down excessive cell division as seen in psoriasis.

### Dithranol mechanism of action and its chemical reactivity (Goranson 1987, Mustakallio 1984, Shroot et al., 1992):

A keynote of the chemical behavior of dithranol is its redox chemistry, an understanding of which highlights the risks and benefits of this drug.. Chemical reactivity of the hydroxyanthrone moiety can be attributed to:
a) phenolic hydroxyl groups at C₁ and C₈
b) two reactive hydrogen atoms at C₁₀

It is observed that during auto-oxidation process, keto-enol tautomerism of dithranol is altered resulting in the formation of 1.8-dihydroxyanthraquinone (danthrone) and 1,8,1',8'-tetrahydroxydianthrane(dianthrone) via the anthronyl radical.

The chemical degradative pathways (or the pathways for anti-psoriatic activity) have been depicted in the following figure:

| | |
|---|---|
| 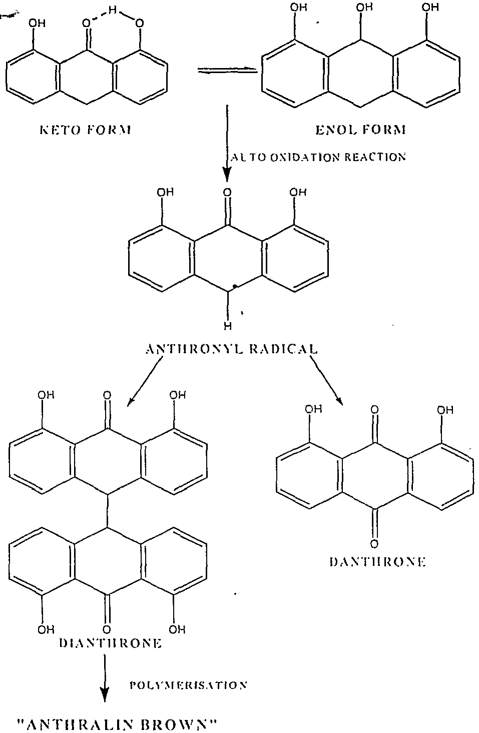 | **PARADOXICAL CHEMICAL BEHAVIOR OF DITHRANOL** |
| | |
| | Auto-oxidative chain reaction of dithranol has two aspects: |
| | **Desirable** as it leads to formation of anthronyl radical which exerts antipsoriatic activity. |
| | **Undesirable** as it leads to formation of danthrone, dianthrone and anthralin brown which are devoid of antipsoriatic activity and are responsible for irritation and staining. |

Oxidation of dithranol is both pH and light dependent. Dithranol displays a distinct chemical behavior involving hydrogen atom abstraction and electron transfer to yield the corresponding anthronyl radical.

It is this chemical reaction (i.e. auto-oxidation), which leads to interference with cellular metabolism that accounts for the ubiquitous pharmacology of dithranol wherein it acts as an electron donor and inhibits the electron transport chain.

Further, the oxidation chain leads to the formation of uncharacterized products known as anthralin brown' or dithranol brown' that are responsible for the violet-blown staining of skin and clothes. All these oxidation products of dithranol (i.e. danthrone, dianthrone and dithranol brown) do not possess antipsoriatic activity and arc responsible for the major side effects associated with dithranol therapy.

Thus, it is a paradoxical situation wherein the chemical reactivity of dithranol is necessary for its antipsoriatic action as an electron generating process, while at the same time it is undesirable as it leads to the formation of oxidized products that are irritating and staining.

### Problems of dithranol, past efforts and reasons for failure (Brandt, Mustakallio, 1983; Loffler et al., 1999, Mustakallio, 1979;, Misch et al., 1981; Paramsothy, Lawrence, 1987)

The problems associated with the dithranol therapy are manifold and are described as below:
- **Irritation -** The dithranol molecule gets quickly oxidized thus yielding free radicals, which damage the biological tissues and irritate profoundly. It is the healthy naïve tissues, which get in contact with dithranol are more irritated than the affected ones.
- **Staining of skin and clothes -** Dithranol causes staining of skin surrounding the lesions, hair and fingernails. It may spoil clothing, linen and bathtubs by permanent staining. Staining is due to destabilization of dithranol molecules, which results in dark brown red color. These oxidized compounds get strongly adsorbed onto the fabric of clothes and are deeply bound to skin tissues. Therefore, the clothes are very difficult to wash and skin remains stained for very long time. This can be ascribed to the physicochemical nature of the decomposed product.
- **Perilesional inflammation -** Inflammatory response to dithranol could be due to an oxidation metabolite. Further, it is also speculated that production of free radicals by dithranol may cause specific peroxidation ofarachidonic acid responsible for the inflammation of skin. And, the vehicles so far used are not able to control the contact between the healthy tissues and decomposition products of dithranol.
- **Itching, stinging, burning and dryness of the skin.**
- **Folliculitis** and **contact allergy** have also been reported.

All these undesirable effects observed during dithranol therapy limits the use of drug and also affect the patient compliance.

Therefore, in the past a lot of efforts have been made in different directions to help manage the dithranol therapy. These include, majorly at the level of:
- **Molecule modification** i.e. chemical change in the molecule to help obtain a stable molecule. But this chemical derivatisation is so far not successful as dithranol is still the most efficacious molecule amongst all of its congeners.
- **Modification in the vehicular composition** by conventional formulation approach and the addition of salicylic acid to help manage stability, solubility and spreadibility.
- **Modification in the treatment modality**

(a) By limiting the time of application (i.e. short contact therapy) and limiting the dose levels.
(b) By applying occlusive covering (plastic film) to help penetrate the molecule.

The above mentioned efforts to improve upon the dithranol molecule per se as well as its formulations may be cited as below:
Dithranol and related hydroxyanthrones chysarobin, triacetyldithranol and 10-butyryldithranol (butantrone) have been compared wherein the modified molecules, namely butantrone and triacetyldithranol have shown reduced irritancy and staining while at the same time their efficacy is simultaneously compromised. Mustakallio, K., K. "The history of dithranol and related hydroxyanthrones, their efficacy, side effects, and different regimens employer in the treatment of psoriasis" Acta Derm Venereol 1992; Suppl. 172: --9.
Various cream formulations of dithranol alone, along with salicylic acid and different stabilizers have been designed with a view to improve stability and efficacy, Kneczke, M.; Rahm, C.; Landersjo, L. and Lundgren, P. "In vitro release of anthralin from white petrolatum and an o/w cream" Acta Pharm. Nord.1989, 1, 249-258. Kneczke, M.: Rahm, C.; Landersjo, L. and Lundgren, P. "The influence of salicylic acid on the in vitro release of anthralin from an o/w cream" Acta. Pharm. Nord.,1990, 2, 313-18. Prins. M.: Swinkels, O.Q.J.: Kolkman, E.G.W.; Wuis, E.W.: Hekster, Y.A. and Van Der Valk. P.G.M. "Skin irritation by dithranol cream. A blind study to assess the role of cream formulation" Acta Derm. Venereol, 1998 (Stockh).. 78, 262-65.

Dithranol has been encapsulated in a matrix of semicrystalline monoglycerides and marketed under the tradename Micanol® Lindahl, A. "Embedding of dithranol in lipid crystals" Acta Derm Venereol 1992: Suppl. 172:13-16.

Mixed vesicular system containing a nanoemulsion with bilayer nanovesicles mimicking niosomes and liposomes (of size 450nm) for topical delivery of dithranol, Gidwani, Suresh Kumar et al., Composition for delivery of dithranol, US patent application 20030219465.

Dithranol (0.5%) has been incorporated in commercially available liposomal gel Natipide® Il and liposomes of size 220nm have been obtained. In this study the penetration of dithranol in the skin has been reported to increase (which is a measure of increased erythema or redness of the skin) with a 10 minute occlusive application of the liposomal gel. The enhanced erythema, after liposomal application, however is unwanted as it may lead to irritancy alongside. Gehring, W.; Ghyczy, M.; Gloor, M.; Scheer, T. and Roding, J. "Enhancement of the penetration of dithranol and increase of effect of dithranol on the skin by liposomes" Arzneim-Forsch. 1992, 42(7), 983-985.

Pharmaceutical preparation containing dithranol have been prepared in a carrier which contains 40 to 60% of liquid paraffin. 40 to 60% of solid paraffin, and 0.5 to 5°n of microcrystalline wax, the composition can be designed into thin, hard and non-brittle sticks for precise application to the site. Mustakallio, et al., US patent no. 4699929.

Dithranol:polyvinylpyrrolidone (Di:PVP) coevaporates have been prepared to increase the aqueous dispersibility of dithranol, these coevaporates contained small crystals as well as nanoparticles of dithranol embedded in the PVP matrix. Delneuville,I.: Dechesne,J.P. and Delattre.L. "Preparation and study of the characteristics of dithranol:polyvinypyrrolidone coevaporates" Int. J. Pharm., 168, 109-118.

Pharmaceutical composition comprising oil in water emulsion containing dithranol and nonionic vesicles prepared from nonionic amphiphilic lipids, in order to prevent the oxidation of dithranol. The vesicles are dispersed in aqueous phase of the emulsion and dithranol is not encapsulated in the vesicles. Laugier, Jean Pierre et al., Pharmaceutical composition for topical application containing dithranol and preparation process. US patent no. 5358716.

A composition containing dithranol, an acid and anti-oxidant, petroleum jelly, which may be mixed with a spreadable synthetic rubber, water, and an emulsifier. This composition shows a reduced staining and little tendency to spread, Yarrow, et al.. USpatent no.4203969.

A liquid preparation of film forming polymers and dithranol, with which the psoriasis affected site can be covered and treated in situ with a medicated film or foil formed at the treatment site. Mueller, et al., US patent no. 4826677.

An anhydrous compositions containing dithranol in a pharmaceutically acceptable anhydrous vehicle (formulated from glyceryl monostearate and isopropyl myristate or isopropyl palmitate), Stable dithranol compositions in anhydrous vehicles, Van Scott et al., US patent no. 4367224.

Pharmaceutical compositions for topical application to the skin containing a dissolved, emulsified or suspended active ingredient (triamcinolone, dithranol, urea or mixture of these) encapsulated in liposomes. The compositions are formulated as ointments or creams, Muller Josef, Topical pharmaceutical compositions, Patent no. ES2002917.

Agarwal et al. (2001) discloses the routine technology to develop dithranol-loaded vesicular carriers, in particular an inter and intralamellar vesicular liposomal preparation with a plurality of concentric bilayered vesicles dispersed therein.

Gehring et al. (1992) discloses the external incorporation of dithranol into a prepared liposomal gel.

US 2002/102298 discloses a temperature-sensitive liposomal formulation for a systemic administration.

EP1364642 discloses an oil and water nano-emulsion containing dithranol in soluble form together with salicylic acid, charcoal and a gelling agent.

WO 94/07466 discloses a liposomal composition entrapping a steroidal antiinflammatory compound and administered intravenously for site specific release.

### Summary of the invention:

In the present invention, it has been identified that stabilization is not the sole factor needed for improvement. It has been discussed earlier that dithranol acts as an electron donor and blocks the electron transport chain in mitochondria of affected cells, thereby bringing about a halt in various energy dependent processes including cell division. So, it can be clearly seen that electron generating capacity i.e. the oxidation of molecule is involved (which is the resultant of dithranol decomposition or instability) in the therapeutic action. Therefore, it can be stated that chemical reactivity (oxidation) of dithranol is rather a required step for its therapeutic action. However, it is known that the oxidation of dithranol results in production of oxidized products, namely, danthrone, dianthrone and anthralin brown, which are responsible for all the side effects associated with dithranol.

It can, therefore, be concluded that the present approach of dithranol stabilization is not altogether correct because stabilizing dithranol molecule means hindering its chemical reactivity (i.e. oxidation process) that will eventually result in lowering down of therapeutic efficacy.

In the light of above paradoxical situation, the degradation of dithranol is desired (for antipsoriatic activity) as well as not desired (for safety and patient compliance). The need of the hour is to moderate the process of degradation in a controlled manner by developing a suitable carrier design composed of appropriate components, which will exert an appropriate (i.e. as and when required) control over dithranol reactivity. The carrier/delivery system should keep dithranol in stable form during storage (i.e. before application to the skin). This will prevent oxidation of dithranol and subsequently the formation of oxidized products that cause irritation and staining (and these oxidized products do not exhibit any antipsoriatic activity). Secondly, after application to skin, the carrier system should result in a controlled release of dithranol to the affected area, which will bring about a sufficient reactivity (i.e. auto-oxidation reaction) in order to exert a desired therapeutic action with minimum of side effects.

The present work was initiated, after identifying the core problems of molecule and the faults in its delivery mechanism, to help evolve some unique strategy, which would allow the drug to locate the site of action in a most wanted fashion, so that the problems of dithranol are minimized and at the same time its efficacy is well preserved or even maximized. And remarkably, the carrier system in its strategic design proved to be successful in bringing about a new potential in this age-old molecule. These carrier systems are basically a liquid crystalline state of the matter, better known as smectic mesophase, which are built on some bio-friendly class II polar compounds (saturated phospholipid molecules) in conjunction with other additives which are supramolecularly arranged to provide the lipid enriched biphasic (aqueous and non-aqueous) state. The drug is dispersed at the molecular level within such an inter and intra multilamellar design. This formulation design is novel in the sense that it provides the desired micro-environment for the drug activity. The microstructured carriers help in the desired delivery of the dithranol molecule to the various psoriatic targets at the inter and intra cellular level as the drug finds an improvised access therein, that allows to accommodate dithranol molecule at the inter and intra compartmental spaces. And, it provides the drug a desired physicochemical cover to that the molecules are dispersed at the molecular level in a biphasic state (i.e. in the hydro-lipophilic ambience). Chemical degradation reaction that generates the free radicals is controlled in this carrier system. This chemical reactivity is responsible for irritant and staining effect of the drug, but at the same time it is desired for therapeutic action that blocks the electron transport chain in the psoriatic cells. The carrier system not only makes the drug safely and effectively delivered but also makes the drug-receptor interaction at various levels of targets (cellular, intercellular and intracellular) as the drug molecules are encaged strategically in the entire system. The carrier also improves the rheological behavior (i.e. thixotropy) of the formulation so that the molecules are confined to the afflicted area and do not spread to normal skin tissues surrounding the psoriatic lesions. The latter minimizes the irritancy and skin staining while the conventional formulations are unable to prevent the contact of dithranol with normal skin, which is the prime cause of these side effects. The carrier also makes the drug easily washable which otherwise gets strongly absorbed onto the fabric and makes the clothes heavily stained (in conventional formulation).

Further, there is observed a synergism in the therapeutic action by way of the formulation components as the dose is reduced significantly i.e. approximately 50%. And the product does not require the incorporation of other frequently used agents like salicylic acid that are known to cause irritation as dithranol alone is sufficiently effective on its own. Thus, there is an overall improvement in the formulation on varied fronts of efficacy and safety, which is worked on the principles of improvised delivery.

The overall improvement in the formulation can be attributed to:

### • Drug Transport

Enhanced drug penetration across the tough and dense lamellar lipidic matrix of stratum corneum is achieved as a result of favorable hydro-lipophilic microenvironment built in by these micro-carrier systems which keep the drug molecule encased within their interior. This is because of the possible carrier-skin lipid interaction that helps in the partitioning of the drug and facilitates the skin transport. The simultaneous modification in the barrier function of stratum corneum due to incorporation of phospholipids (into skin layers) also favors the transport of drug across skin.

### • Control on release, reactivity and stability

Phospholipid molecules known for the anti-oxidant activity provide a supramolecular cover to the highly unstable dithranol molecule that is placed within the system. The drug molecule remains protected (this feature is responsible for improved shelf-life or storage life of the drug) but when they arc applied, release at a controlled rate from the intra-lamellar domain, and tend to auto oxidized along with the molecules that are already present at the inter-lamellar space. This results in controlled oxidation and sufficient electrons are generated for the desired action on psoriatic targets (so that the electron transport chain is blocked and cell division and subsequent proliferation is restricted). The rate of release of drug from interior compartments is well controlled by way of multilamellar membrane barrier. So, in totality the drug molecule placed at inter and intra compartmental sites show moderation in reactivity to obtain improved safety as well as efficacy.

### • Super-solvent effect

Phospholipid-based carrier systems by virtue of the amphiphilicity and supramolecular association provide a super solvent effect, which keeps the active medicament in molecularly dispersed state so that the transfer across the skin is facilitated.

### • Prolonged and pronounced local action

Increased drug-localization in the skin strata is expected by the formation of micro-reservoirs, which results in confinement and retention of drug within affected skin tissues. This helps to produce prolonged local action and reduced side effects. Besides prolonged presence, the pronouneed action is another benefit that may be attributed to the presence of the therapeutic molecules at or near the target site. The drug in the carrier may produce better drug-target interactions.

### • Dose reduction & improved therapeutic index

The above-mentioned points conglomerate to lead to a reduction in the dose level to almost half of the dose of conventional preparation (i.e. 0.5% w/w vis-a-vis 1.15% w w of conventional preparation). This is a substantial enhancement in the therapeutic index of the drug. So, the safety of the drug is also expected to be increased, because an enhanced drug action is obtained, as a lesser number of molecules are required to elicit the desired pharmacotherapeutic effect. And also the hydrating effect in the lipid rich environment also reduces the irritant effect of the molecule.

### • Skin nutrition

Phospholipids carrier-based product provides the desired skin moisttirizing and emollient effect. Phospholipid enriched system (i.e. multilamellar system with lamellac comprising of phospholipid molecules *vis-à-vis* unilamellar systems) add value to the product as the skin is fed with the desired membranous lipids that too in a hydrated state.

### • Improved patient compliance

Reduced irritancy and staining along with favorable rheological properties of the system in contrast to greasy ointments make it cosmetically more acceptable and improve the patient compliance..

### • Less staining and easily washable

Dithranol molecule being highly lipophilic gets deeply bound to skin tissues and the fabric of the clothes thus making it very difficult to wash out, while dithranol micro enclosed in this system gets a lesser chance of direct interaction with skin or fabric. Also, this system being surface active in nature docs not allow dithranol to be strongly hound to these surfaces, thus making it easy to wash off with warm water without any need of detergent. Moreover, the aqueous gel base forms the external vehicle of the product that makes the preparation easily washable from skin as well as clothing.

The present invention could peep deep down into the troubles of dithranol and diagnose the desired delivery dimensions of the drugs that remained hitherto not focused properly. However, few products have been made available in the market (e.g. Micanol) based on some entrapment strategy and could deal with some aspects of dithranol delivery but none could result into desired success which will take care of the drug in toto.

The present product has made success, after identifying the problem in right form and projecting the hypothesis accordingly, by entrapping the drug at the intra and inter-lamellar microstructured domains composed of most conducive molecular compositions at the supramolecular level.

The strategically composed systems with carrier effect could help modify the various chemical and physicochemical characteristics of the drug such as reactivity, release, solubility, stability and spreadibility that in turn end up with a unique formulation which is improved on its shelf-life, efficacy, safety (non-irritating), patient compliance (non-staining on clothes and skin), rheology (thixotropic) and cosmetic value. The formulation so developed is not only effective at the reduced dose level but now can bear enhanced dose level that is desired in certain types of psoriasis and also can be left in contact with the affected site for a prolonged period of time (in contrast to presently practised short contact therapy). Further, the formulation docs not need any additional support such as that of salicylic acid (an irritant molecule) and occlusive plastic covering onto the affected site.

### Detailed description of the invention:

The drug along with the lipid components namely, phospholipid, cholesterol and butylaled hydroxyl toluene are dissolved in sufficient amount of chloroform in a round bottom flask. The flask is then attached to the rotavapor for evaporation of the solvent and formation of a thin film. The flask is subjected to overnight vacuum for complete removel of the residual solvent. The dry, thin film is then hydrated with phosphate bettered saline of pH 6.4 containing sodium metabisulfite. The suspension so obtained is kept for separation of layers and the supernatant is remove so as to adjust the final concentration of the product. The suspension is added to the required amount of methylcellulose so as to gel the entire system. The addition of methylcellulose is a crucial step, wherein methylcellulose is made into slurry at high temperature (60 to 65"C) and then added two the entire system at a substantially lower temperature (0 to 5°C). This transition in temperature allows to form a gel product in a quicker succession so as to encage the vesicles within the gel matrix to help maintain the uniformity. The various parameter which influence the quality of the product include the level of the drug, lipid, solvent, nature of the hydration medium, hydration temperature and the process variables such as rotational speed, temperature and the surface area of the glass support etc.

The maximum amount of drug loaded into vesicles was observed to be 3.51 mag/185 mag of total lipid four a maximum degree of entrapment for maintaining the size uniformity along with freedom from aggregation.

The mean size, using laser light scattering technique for dithranol vesicles was found to be 3.8±0.4µ. With optical microscopy the mean size (dᵥₙ) of vesicles was found to be 3.59 ± 1.5 µ. The size of the vesicles is subjected to mechanical perturbations and can be varied by employing mechanisms such as vortexing and ultrasonication. However, it is initially kept at this level make the micro-carrier lipid enriched system for better drug delivery.

The leakage of drug was recorded to be increased (≈40%) at higher temperature (37°C). While at 4°C and 25°C vesicles have shown fairly high retention of drug, i.e. minimum leakage (≈ 10%) inside the vesicles up to a period of one month (≈ 90%).

A 2% methylcellulose gel with a pH maintained at 6.0=0.5 finally found suitable, the concentration of methylcellulose may vary from 1% to 5% depending on the viscosity grade. The vesicles incorporated in the gel were observed to be well preserved as monitored by optical microscopy. The rheological profile of the final formulation was determined and found to possess the thixotropic tendency. The latter makes the product easily applicable (owing to improved spreadibility) onto the afflicted areas but after the application does not allow it to spread to naïve tissues surrounding the afflicted site, which is the main cause of irritation and staining.

The final composition after addition of secondary vehicle, methylcellulose (2%), contains 0.5% w/w dithranol which includes 70.2% intravesicular drug and 29.8% intervesicular drug. The gelling agent, methylcellulose is a hydrocolloid which can be replaced by other such gel forming agents e.g. hydroxypropylmethylcellulose, carbopol, hydroxyethylcellulose, sodium carboxymethylcellulose, gelatin etc.

The vesicular systems are release rate retarding by virtue of their lamellar barrier effect. The release of dithranol is retarded up to 9.4µg/cm²/h from intra-lamellar spaces of the vesicles whilst in case of solution it was at much higher level of release rate 42.9 µg/cm²/h. This implies that the release retarding nature of the system is helpful in controlling the activity of the drug onto the site(s) of action. Also, this retarding nature helps in obtaining the depot effect so as to prolong the action of drug, which can be seen in the following observation.

The skin retention with the formulation was observed to be 0.45 mg/4.15cm² skin area, while in case of solution it was only 0.058 mg /4.15cm² skin area.

The vesicular preparation could protect the dithranol to the extent of 91.87% in the storage conditions, 40°C 75% RH with light for a 3-month period while the conventional cream could protect up to 73.24%. In the stabilization, the type of phospholipid used played a major role while the antioxidant and preservative gave a support. The presence of a large concentration of the phospholipid molecules, which are inherently anti-oxidant in nature helps to protect the drug from oxidative degradation during the storage conditions. The drug is also protected by virtue of its position in the system composed of biphasic i.e. aqueous and non-aqueous phases where the aqueous phase is a hydrogel system and not a simple aqueous solution.

The highly stained clothes with commercial formulation were difficult to washout even with detergent. However, the vesicular systems were free from this discoloration problem and whatever little marks they left were easily washed out. This is because the lipidic bilayer prevents the direct contact of the naked drug molecule with the fabric. thereby resulting in a negligible adsorption on the cloth surface, while in the conventional formulations the drug gets highly bound to the fabric and stains it permanently.

The observations made during irritancy studies indicate that dithfanol in vesicular product has been devoid of the skin irritancy effect. The conventional and Commercial cream have shown a significant skin-worsening effect to a extent that application could not be continued for a stipulated time period of study.

The vesicular product was compared with Commercial formulation while taking plain vesicular gel (without drug) as its control. The dithranol-vesicular gel formulation (0.5%) has been found to have comparable efficacy to that of commercially available dithranol cream (1.15°o). At the end of study (as reflected in the total severity score [TSS] value) the reduction in TSS by dithranol vesicular gel is noted to be slightly higher (1.6±0.2), than that of commercial cream A (1.5±0.2) but not significant. The performance of vesicular gel in its drug content level of 0.5% vis a vis 1.15% of commercial cream is adjudged as enhanced. On the issue of safety and acceptability of the dithranol formulation, vesicular product could perform much better than the commercial cream. The level of adverse effects (irritation, perilesional erythema, burning sensation and staining of skin and clothes) was reported to be very low in comparison to commercial one. Also the acceptability of the patients was of high order that make the product favorable by physician who was carrying the trial.

Another important difference of the intra and inter vesicular product with respect to the conventional product is that it does not require the incorporation of salicylic acid for stability or keratolytic activity. Salicylic acid is a known irritant which is otherwise present in most of the conventional products. But, here in this product the composition and its design strategy helps to do away with such irritants so as to make the product more patient friendly.

Thus, the improved clinical performance of dithranol in dithranol vesicles is concludingly held responsible for their various compositional factors and working mechanisms on and within the affected skin site. Now with this finding the dithranol can be seen to gain a better potential and image so that its vesicular form can be allowed to treat sensitive skin areas like face and flexures. Also, larger body surface areas (including soft tissues) may be safely treated without fear of severe irritation.

Inter and intra multilamellar vesicular composition of the instant invention containing dithranol includes one or more other antipsoriatic, keratolytic or immunoinflammatory agents.

### Example

### Composition of the prepared dithranol liposomal product:

| **S.No.** | **Name of the ingredient** | **Amount of ingredient per 20gm of the product** | **% w/w** |
|---|---|---|---|
| **1** | Dithranol | 100 mg | 0.5 |
| **2** | Phospholipid | 2.0 gm | 10.0 |
| **3** | Cholesterol | 1.0 gm | 5.0 |
| **4** | Butylated Hydroxy Toluene (Antioxidant) | 60.0 mg | 0.3 |
| **5** | Methylcellulose | 400mg | 1.0-2.0 |
| **6** | Phosphate Saline Buffer USP pH 6.4 containing 0.5%w/v Sodium metabisulphite (Antioxidant) | q.s. 20.0 gm | |

| **Parameter** | **Value** |
|---|---|
| **Dithranol per total product** | 0.5%w/w |
| **Dithranol:Total lipids (weight ratio)** | 100mg drug : 3000mg total lipids |
| **% Dithranol per Total lipids** | 3.33% of total lipids |
| **Dithranol Intralamellar (intravesicular)** | 70.2% of the total drug added |
| **Dithranol Interlamellar (intervesicular)** | 29.8% of the total drug added |
| **Dithranol Intralamellar (intravesicular) with respect to total lipids** | 56.919 % (56.919 mg out of 100 mg) |
| **Dithranol Interlamellar (intervesicular) with respect to total lipids** | 43.081 % (43.081 mg out of 100 mg) |

### Brief description of the prepared product

| **S. No.** | **Parameter** | | | **Value** | | |
|---|---|---|---|---|---|---|
| **1** | **Drug Entrapment** | | | 3.51 mg/185 mg of total lipids | | |
| **2** | **Mean Size of vesicles** | | | 3.8±0.4µ. | | |
| **3** | **Drug retention at 4°C and 25°C** | | | ≈ 90% Up to a period of one month | | |
| **4** | **pH of the vesicles incorporated in 2% secondary vehicle (i.e. 2.0 % methylcellulose gel)** | | | 6.0±0.5 | | |
| **5** | **Rheological behaviour of the product** | | | Thixotropic | | |
| **6** | **In-vitro release rate** | | | | | |
| | **• Vesicles in MC gel** | | | 9.4µg/cm²/h | | |
| | **• Drug solution** | | | 42.9 µg/cm²/h | | |
| **7** | **In-vitro skin permeation rate** | | | 23.9 µg/cm²/h | | |
| | **• Vesicles in MC gel** | | | 36.9 µg/cm²/h | | |
| | **• Drug solution** | | | | | |
| **8** | **Retention of drug in Skin** | | | | | |
| | **• Vesicles in MC gel** | | | 0.48 mg /4.15 cm² | | |
| | **• Drug solution** | | | 0.058 mg 4.15 cm² | | |
| 9 | **Stability data (Assay of dithranol)** | | | | | |
| | | **Vesicles** | **4°C** | **40°C/75% RH** | **40°C75% RH with light** | |
| | | 1 month | 99.51 ± 1.05 | 99.42 ± 1.15 | 98.11 ± 0.65 | |
| | | 2 month | 99.23 ± 1.8 | 99.12 ± 1.42 | 95.23 ± 0.92 | |
| | | 3 month | 99.61 ± 1.2 | 99.05 ± 1.1 | 91.87 ± 0.78 | |
| | | **Cream** | **4°C** | **40°C/75% RH** | **40°C/75% RH with light** | |
| | | 1 month | 96.87 ± 0.75 | 95.89 ± 1.2 | 89.78 ± 0.91 | |
| | | 2 month | 95.78 ± 0.5 | 93.94 ± 0.85 | 80.12 ± 0.77 | |
| | | 3 month | 94.12 ± 1.1 | 92.11 ± 0.9 | 73.24 ± 0.63 | |
| **10** | **Staining properties (Washability)** | | | Easy to wash and free from staining problem Difficult to washout even with detergent | | |
| | **Vesicular product** | | | | | |
| | **Conventional cream** | | | | | |
| **11** | **Skin irritancy effect** | | | Free from irritancy effect Shows a significant skin-worsening | | |
| | **• Dithranol in vesicles** | | | | | |
| | **• Conventional and commercial cream** | | | | | |
| **12** | **Clinical efficacy and safety of the product vis-a-vis conventiona cream** | | | **Efficacy** | | |
| | | | | • The dithranol-vesicular gel formulation (0.5%) have comparable efficacy to that of commercially available dithranol cream (1.15%). | | |
| | | | | | | |
| | | | | **Total severity score [TSS] value:** | | |
| | | | | • Dithranol vesicular gel: 1.6±0.2 | | |
| | | | | • Commercial cream A: 1.5±0.2 (but not significant) | | |
| | | | | | | |
| | | | | **Safety and Acceptability of the vesicular formulation** | | |
| | | | | • Vesicular product had performed much better than the commercial cream. | | |
| | | | | • The level of adverse effects (irritation. perilesional erythema, burning sensation and staining of skin and clothes) was reported to be very low in comparison to commercial one. | | |
| | | | | • Also the acceptability of the patients was of high order that make the product favorable by physician who was carrying the trial. | | |

## Claims

1. A controlled release drug delivery system having a dispersion of inter and intra multilamellar vesicular composition for use in the treatment of dermal problems such as psoriasis, said system comprising
a biphasic arrangement such that the outer phase comprises an aqueous hydrocolloidal gelling agent which constitutes the interlamellar phase and an inner phase which comprises multiple lipoidal vesicles constituting the intralamellar phase,
such that the drug contained in said vesicular composition disperses into both the phases in critically defined concentrations of 70% to 80% in the said inner phase and 20% to 30% in the said outer phase.

2. The drug delivery system of claim 1, wherein lipoidal vesicles have the composition comprising
| **Ingredient %** | **w/w** |
|---|---|
| dithranol | 0.25 to 1.0 |
| phospholipid | 8 to 12 |
| cholesterol | 3 to 7 |
| butylated hydroxy toluene | 0.1 to 0.5 |
| hydrocolloid gelling agent | 0.5 to 3.0 |
dispersed in 0.2 Molar phosphate Saline Buffer USP pH 6.4.

3. The drug delivery system of claim 1, wherein the average size of the said lipoidal vesicles ranges from 1 µm to 10 µm.

4. The drug delivery system of claim 1, wherein the said hydrocolloid gelling agent is methylcellulose.

5. The drug delivery system of claim 1, wherein the drug is available to the skin cells in a reduced but continuous manner owing to diffusion from inner lipoid vesicles to the outer aqueous phase as degradation proceeds in aqueous phase.

6. The drug delivery system of claim 1, wherein dispersal of the drug in defined concentrations in the aqueous and non-aqueous phases permits controlled degradation of dithranol leading to free radical generation required for therapeutic effect.

7. The composition of claim 1 comprising a novel controlled release drug delivery system wherein said hydrocolloid gel possesses the thixotropic rheological character.

## Patentansprüche

1. Arzneistoffabgabesystem zur kontrollierten Freisetzung mit Dispersion von inter- und intramultilamellarer vesikulärer Zusammensetzung zur Verwendung bei der Behandlung von dermalen Problemen wie Psoriasis, wobei das System umfasst:
eine biphasische Anordnung, so dass die äußere Phase ein wäßriges hydrokolloidales Geliermittel umfasst, das die interlamellare Phase darstellt, und eine innere Phase, die multiple lipoidale Vesikel umfasst, die die intralamellare Phase darstellen,
so dass sich der Arzneistoff, der in der vesikulären Zusammensetzung enthalten ist, in beide Phasen bei genau definierten Konzentrationen von 70% bis 80% in der inneren Phase und 20% bis 30% in der äußeren Phase verteilt.

2. Arzneistoffabgabesystem nach Anspruch 1, wobei die lipoidalen Vesikel die Zusammensetzung haben, die umfasst:
| **Inhaltsstoff %** | **Gew./Gew.** |
|---|---|
| Dithranol | 0,25 bis 1,0 |
| Phospholipid | 8 bis 12 |
| Cholesterol | 3 bis 7 |
| butyliertes Hydroxytoluol | 0,1 bis 0,5 |
| Hydrokolloid-Geliermittel | 0,5 bis 3,0 |
dispergiert in 0,2 molarer phosphatgepufferter Kochsalzlösung USP pH 6,4.

3. Arzneistoffabgabesystem nach Anspruch 1, wobei die Durchschnittsgröße der lipoidalen Vesikel im Bereich von 1 µm bis 10 µm liegt.

4. Arzneistoffabgabesystem nach Anspruch 1, wobei das Hydrokolloid-Geliermittel Methylcelullose ist.

5. Arzneistoffabgabesystem nach Anspruch 1, wobei der Arzneistoff für die Hautzellen in einer verringerten jedoch kontinuierlichen Weise verfügbar ist aufgrund der Diffusion von inneren lipoidalen Vesikeln zur äußeren wässrigen Phase, während der Abbau in der wässrigen Phase fortschreitet.

6. Arzneistoffabgabesystem nach Anspruch 1, wobei die Verteilung des Arzneistoffs bei definierten Konzentrationen in der wässrigen Phase und der nicht-wässrigen Phase einen kontrollierten Abbau von Dithranol ermöglicht, was zur Erzeugung von freien Radikalen führt, die für die therapeutische Wirkung notwendig sind.

7. Zusammensetzung nach Anspruch 1, die ein neues Arzneistoffabgabesystem zur kontrollierten Freisetzung umfasst, wobei das Hydrokolloid-Gel thixotrope rheologische Eigenschaften hat.

## Revendications

1. Système de délivrance de médicament à libération contrôlée comprenant une dispersion de composition vésiculaire inter et intra multilamellaire destiné au traitement de problèmes cutanés tels qu'un psoriasis, ledit système comprenant
une organisation biphasique de telle sorte que la phase extérieure comprend un agent gélifiant hydrocolloïde aqueux lequel constitue la phase interlamellaire et une phase intérieure laquelle comprend de multiples vésicules lipoïdes constituant la phase intralamellaire,
de telle sorte que le médicament contenu dans ladite composition vésiculaire se disperse dans les deux phases selon des concentrations critiques définies comprises entre 70 % et 80 % dans ladite phase intérieure et entre 20 % et 30 % dans ladite phase extérieure.

2. Système de délivrance de médicament selon la revendication 1, dans lequel des vésicules lipoïdes présentent la composition comprenant
| **Ingrédient %** | **m/m** |
|---|---|
| dithranol | de 0,25 à 1,0 |
| phospholipide | de 8 à 12 |
| cholestérol | de 3 à 7 |
| hydroxytoluène butylé | de 0,1 à 0,5 |
| agent gélifiant hydrocolloïde | de 0,5 à 3,0 |
dispersés dans un tampon phosphate salin de qualité USP (pharmacopée des Etats-Unis) à 0,2 M et à pH 6,4.

3. Système de délivrance de médicament selon la revendication 1, dans lequel la taille moyenne desdites vésicules lipoïdes est comprise entre 1 µm et 10 µm.

4. Système de délivrance de médicament selon la revendication 1, dans lequel ledit agent gélifiant hydrocolloïde est la méthylcellulose.

5. Système de délivrance de médicament selon la revendication 1, dans lequel le médicament est disponible pour les cellules cutanées de manière réduite mais continue grâce à une diffusion depuis les vésicules lipoïdes intérieures vers la phase aqueuse extérieure à mesure qu'une dégradation a lieu en phase aqueuse.

6. Système de délivrance de médicament selon la revendication 1, dans lequel une dispersion du médicament selon des concentrations définies dans les phases aqueuse et non aqueuse permet une dégradation contrôlée de dithranol conduisant à une formation de radicaux libres nécessaires pour un effet thérapeutique.

7. Composition selon la revendication 1, comprenant un nouveau système de délivrance de médicament à libération contrôlée, dans laquelle ledit gel hydrocolloïde possède le caractère rhéologique thixotropique.
